(19) Europäisches Patentamt
European Patent Office
Office européen des brevets

(11) Veröffentlichungsnummer: **0 289 786 B1**

# (12) EUROPÄISCHE PATENTSCHRIFT

(45) Veröffentlichungstag der Patentschrift: **08.12.93**

(51) Int. Cl.$^5$: **G02F 1/137**

(21) Anmeldenummer: **88105353.2**

(22) Anmeldetag: **02.04.88**

## (54) Verwendung von Verbindungen oder Gemischen von Verbindungen, die eine chirale, orthogonale, höher geordnete smektische Phase aufweisen, im Bereich dieser Phase als Schalt- oder Anzeigemedium.

(30) Priorität: **07.04.87 DE 3711360**
**29.05.87 DE 3718174**

(43) Veröffentlichungstag der Anmeldung:
**09.11.88 Patentblatt 88/45**

(45) Bekanntmachung des Hinweises auf die Patenterteilung:
**08.12.93 Patentblatt 93/49**

(84) Benannte Vertragsstaaten:
**AT BE CH DE ES FR GB IT LI NL SE**

(56) Entgegenhaltungen:

**Proceedings of the Fifth European winter Liquid Crystal Conference PART B Borovets Bulgaria March 25-30, 1987 Published in Molecular Crystals and Liquid Crystals vol 152 (1987), pages 528-533, Sven T. Lagerwall "Material properties of ferroelectric Liquid Crystals and their relevance in applications and devices"**

(73) Patentinhaber: **HOECHST AKTIENGESELLSCHAFT**

**D-65926 Frankturt(DE)**

(72) Erfinder: **Bahr, Christian**
**Hohenzollerndamm 189**
**D-1000 Berlin 31(DE)**
Erfinder: **Heppke, Gerd, Prof. Dr.**
**Johann-Georg-Strasse 3**
**D-1000 Berlin 31(DE)**
Erfinder: **Lötzsch, Detlef**
**Lessingstrasse 10**
**D-1000 Berlin 21(DE)**
Erfinder: **Dübal, Hans-Rolf, Dr.**
**Heuhohlweg 6**
**D-6240 Königstein/Taunus(DE)**
Erfinder: **Escher, Claus, Dr.**
**Amselweg 3**
**D-6109 Mühltal(DE)**
Erfinder: **Ohlendorf, Dieter, Dr.**
**Am Kühlen Grund 4**
**D-6237 Liederbach(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 289 786 B1

## Beschreibung

Die ungewöhnliche Kombination von anisotropen und fluiden Eigenschaften der Flüssigkristalle haben zu ihrer Verwendung in einer Vielzahl von elektro-optischen Schalt- und Anzeigevorrichtungen geführt. Dabei können ihre elektrischen, magnetischen, elastischen oder thermischen Eigenschaften zu Orientierungsänderungen benutzt werden. Optische Effekte lassen sich dann mit Hilfe ihrer Doppelbrechung ("birefringence mode"), der Einlagerung dichroitisch absorbierender Farbstoffmoleküle ("guest-host mode") oder der Lichtstreuung erzielen.

Dabei wurden bisher, von wenigen Ausnahmen abgesehen, die nematische Phase (N) und die smektischen Hochtemperaturphasen $S_A$ und $S_C$, bzw. ihre chiralen Versionen N*, $S_A^*$ und $S_C^*$ verwendet.

Die $S_A$ und $S_C$-Phasen besitzen eine Schichtenstruktur mit statistisch verteilten Molekülschwerpunkten innerhalb einer Schicht. Sie unterscheiden sich dadurch, daß in der $S_A$-Phase der Direktor $\hat{n}$ senkrecht zur Schichtenebene, d.h. parallel zur Schichtennormale $\hat{z}$ steht (Definition der orthogonalen Phasen), in der $S_C$-Phase jedoch eine Neigung vorliegt, die durch den Winkel $\theta$ zwischen $\hat{n}$ und $\hat{z}$ angegeben wird (Definition der geneigten Phasen oder "tilted phases").

In der von Clark und Lagerwall [N.A. Clark und S.T. Lagerwall, Appl. Phys. Lett. 36, 899 (1980)] vorgeschlagenen "bookshelf"-Geometrie läßt sich die Ferroelektrizität der $S_C^*$ Phase zu einem elektro-optischen Effekt ausnutzen. Dieser beruht auf dem Vorliegen zweier stabiler Zustande, zwischen denen ein schnelles Schalten in typischerweise 50 $\mu$s bei einem elektrischen Feld von $10^7$ V/m erfolgt (R.B. Meyer, L. Liebert, L. Strzlecki und P. Keller, I. Phys. (Paris) Letters 36, L-69 (1975)).

Dieser ferroelektrische Effekt ist durch eine stark nichtlineare elektro-optische Kennlinie ausgezeichnet.

Es ist bekannt, daß in der $S_A^*$ -Phase (chirale $S_A$-Phase) ein verwandter, jedoch linearer Prozeß abläuft, der nach Garoff und Meyer (S. Garoff und R.B. Meyer, Phys. Rev. Lett. 38, 848 (1977)) der elektrokline Effekt heißt. Er beruht auf einem feldinduzierten und dem parallel zu den smektischen Schichten verlaufenden elektrischen Feld E proportionalen Neigungswinkel $\theta$ in der an sich orthogonalen $S_A^*$ -Phase. Die Größe des elektroklinen Effektes wird durch den Differentialkoeffizienten ($d\theta/dE$) angegeben. In den höher geordneten Phasen, z.B. $S_B$, $S_E$ usw., sind die Molekülschwerpunkte innerhalb einer Schicht nicht statistisch verteilt, sondern regelmäßig, ähnlich wie in einem Kristallgitter, angeordnet. Dieser höheren Ordnung entspricht eine höhere Viskosität und Festigkeit der Schichten. Für den Einsatz in elektro-optischen Bauteilen, bei denen schnelle Reaktionen der Flüssigkristallschichten auf Änderungen eines angelegten elektrischen Feldes verlangt werden, wurden daher die höher geordneten smektischen Phasen, insbesondere die orthogonalen $S_B$ uns $S_E$ Phasen nicht in Betracht gezogen.

Es wurde nun überraschenderweise gefunden, daß $S_B^*$ (chirale $S_B$) Phasen und $S_E^*$ (chirale $S_E$) Phasen einen elektroklinen Effekt mit unerwartet kurzen Ansprechzeiten bis herab zu 1,5 $\mu$s zeigen, wobei außerdem der elektrokline Koeffizient $d\theta/dE$ größer ist als in der $S_A^*$ Phase.

Gegenstand der Erfindung ist daher die Verwendung von Verbindungen oder Gemischen von Verbindungen, die eine chirale, orthogonale, smektische Phase, gemäß dem Anspruch 1.

Gegenstand der Erfindung ist weiterhin insbesondere die Verwendung solcher Verbindungen oder Gemische von Verbindungen in Schalt- und Anzeigeelementen aus der Gruppe

a) elliptische Polarisatoren
b) Lichtmodulatoren in "birefringence mode"
c) Lichtmodulatoren in "guest-host mode"
d) Pulsschneider
e) Grauskalen für Bildschirme und Drucker
f) elektrisch gesteuerte Matrices für nichtlinear optische Materialien und
g) Sensoren für elektrische Felder

und Schalt- und Anzeigeelemente, die die genannten Verbindungen oder Gemische von Verbindungen enthalten.

Durch die Erfindung wird eine große Zahl von Verbindungen, die bisher nur theoretisches Interesse hatte, für die Verwendung in elektro-optischen Bauelementen verfügbar. So weisen etwa 25 % der in dem Tabellenwerk "Flüssige Kristalle in Tabellen", Demus et al., VEB Deutscher Verlag für Grundstoffindustrie, Leipzig 1974, verzeichneten smektischen Substanzen eine $S_B$ oder $S_E$ Phase auf. Soweit es sich dabei nicht um chirale Verbindungen handelt, kann die Chiralität durch den Zusatz chiraler Dotierstoffe induziert werden.

Zu den geeigneten Verbindungen in der erfindungsgemäßen Verwendung zählen grundsätzlich solche, die smektogen sind, d.h. eine Neigung zur Bildung smektischer Phasen haben, wobei erfindungsgemäß diese smektische Phase dieser Verbindungen und Gemische von Verbindungen noch chiral, orthogonal und höher-geordnet smektisch sein muß. Beispiele für Verbindungen dieses Typs gehören insbesondere zu den

Verbindungen der allgemeinen Formel (A)

$R^1(-A^1)_j(-M^1)_k(-A^2)_l(-M^2)_m(-A^3)_n-R^2$ (A)

in der bedeuten:

$R^1$,$R^2$ unabhängig voneinander geradkettiges oder verzweigtes (mit oder ohne asymmetrisches C-Atom) Alkyl oder Alkenyl mit 2 bis 16 C-Atomen, wobei auch eine oder zwei nicht-benachbarte $-CH_2$-Gruppen durch -O-, -S-, -CO-, -CO-O-,-O-CO- oder -O-CO-O- ersetzt sein können und wobei auch H durch F ersetzt sein kann. oder einer der nachfolgenden Reste

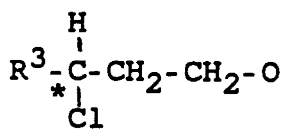
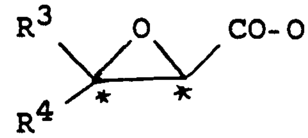

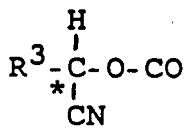
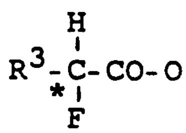
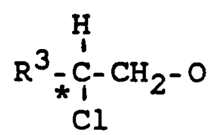

$A^1$,$A^2$,$A^3$ unabhängig voneinander 1,4-Phenylen, trans-1,4-Cyclohexylen, Pyrazin-2,5-diyl, Pyridazin-3,6-diyl, Pyridin-2,5-diyl, Pyrimidin-2,5-diyl, 1,3,4-Thiadiazol-2,5-diyl

$M^1$,$M^2$ unabhängig voneinander CO-O, O-CO, CO-S, S-CO, $CH_2O$, $OCH_2$, CH = N, N = CH, $CH_2$-$CH_2$, N = N, N = N(O)

$R^3$,$R^4$ unabhängig voneinander H oder geradkettiges oder verzweigtes Alkyl mit 1 bis 16 oder Alkenyl mit 2 bis 16 C-Atomen, bei dem auch eine $-CH_2$-Gruppe durch -O-, -CO-O- oder -O-CO- ersetzt sein kann,

j,k,l,m,n Null oder 1, wobei j + l + n = 2 oder 3 ist.

Die Verbindungen sind für den Fachmann auf dem Flüssigkristallgebiet literaturbekannt (siehe z.B. vorstehendes Tabellenwerk) bzw. werden erstmals in der DE-A 37 18 174 beschrieben (für die Oxiranderivate).

Der elektrokline Effekt in höher geordneten smektischen Phasen wird sowohl an zwei optisch-aktiven Einzelverbindungen, nämlich:

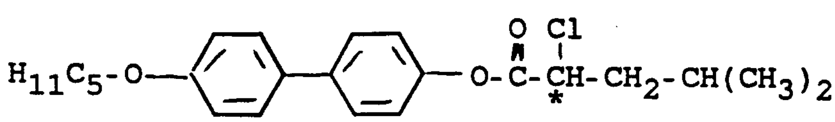
(I)

Phasenfolge K* 58 $S_E^*$ 63,5 $S_B^*$ 72,5 $S_A^*$ 75 I*

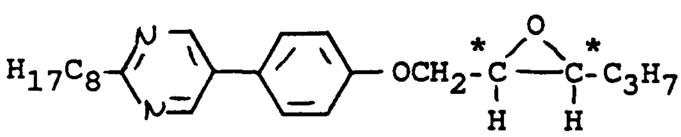
(II)

Phasenfolge K* 55 $S_B^*$ 90 $S_A^*$ 102 I*, als auch an einigen Mischungen demonstriert. Zur Messung der Kenngrößen wurde eine Meßzelle mit einer Schichtdicke der zu messenden Substanz von 2 $\mu$m verwendet. Die planare Orientierung ("bookshelf"-Geometrie) wurde durch Scherung und/oder Orientierungsschichten erhalten. Der Aufbau der Meßzelle ist für ferroelektrische Flüssigkristallschichten von Skarp und Andersson beschrieben (K. Skarp und G. Andersson, Ferroelectrics Letters 6, 67 (1986)).

Der elektrokline Koeffizient $d\theta/dE$ wurde durch Anlegen einer Gleichspannung und optische Bestimmung des Tiltwinkels $\theta$ anhand der Dunkelstellung zwischen gekreuzten Polfiltern relativ zur Nullfeldlage im Polarisationsmikroskop gemessen.

Die elektrokine Schaltzeit wurde bestimmt, indem anhand der Frequenzabhängigkeit der Dielektrizitätkonstanten, die Grenzfrequenz $f_G$ bestimmt wurde, bis zu der die Reorientierung der Moleküle dem Wechselfeld noch folgen kann. Der Reziprokwert dieser Frequenz ist die minimale elektrokline Schaltzeit.

Die mit den Verbindungen (I) und (II) erhaltenen Meßwerte sind in den Tabellen 1 und 2, die Mischungsergebnisse in Tabelle 3 dargestellt. Bei Verbindung (I) sieht man, daß der elektrokline Koeffizient $d\theta/dE$ in den höher geordneten $S_E^*$ bzw. $S_B^*$ Phasen signifikant größer ist als in der $S_A^*$ Phase. Bei der Verbindung (II) wurde in der $S_A^*$ Phase kein meßbarer elektrokliner Effekt gefunden. Wie sehr der elektrokline Koeffizient durch geeignete Dotierstoffe erhöht werden kann, zeigt das Mischungsbeispiel M1.

Tabelle 1: **Meßwerte für die Verbindung (I)**

| t (°C) | Phase - | $\frac{d\theta}{dE}$ ($10^{-9}$ rad m/V) | $\theta$ bei $10^7$ V/m (Grad) | $f_G$ (KHz) |
|---|---|---|---|---|
| 62,5 | $S_E^*$ | 5,0 | 2,8 | 2,5 |
| 64,3 | $S_B^*$ | 7,0 | 4,0 | 36 |
| 70,5 | $S_B^*$ | 5,3 | 3,0 | 230 |
| 72,1 | $S_B^*$ | 5,2 | 3,0 | 630 |
| 72,5 | $S_A^*$ | 3,8 | 2,2 | > 3000 |

Tabelle 2: Meßwerte für die Verbindung (II)

| t (°C) | Phase - | $\frac{d\theta}{dE}$ ($10^{-9}$ rad m/V) | θ bei $10^7$ V/m (Grad) |
|---|---|---|---|
| 60 | $S_B^*$ | 2,0 | 1,2 |
| 70 | $S_B^*$ | 1,6 | 1,0 |
| 80 | $S_B^*$ | 1,1 | 0,6 |
| 95 | $S_A^*$ | kein eletrokliner Effekt beobachtet. | |
| 100 | $S_A^*$ | | |

Tabelle 3: Meßwerte für die Mischungen

| Mischung | Phase | t (°C) | $\frac{d\theta}{dE}$ ($10^{-9}$ rad m/V) | θ bei $10^7$ V/m (Grad) |
|---|---|---|---|---|
| M1 a) | $S_A^*$ | 72 | kein elektrokliner Effekt beobachtet | |
| | $S_B^*$ | 69 | elektrokliner Effekt optisch nachgewiesen | |
| | $S_B^*$ | 60 | 0,9 | 0,5 |
| | $S_B^*$ | 50 | 1,4 | 0,8 |
| | $S_B^*$ | 40 | 2,3 | 1,3 |
| | $S_B^*$ | 36 | 3,5 | 2,0 |
| M2 b) | $S_B^*$ | 50 | 0,6 | 0,35 |

| | | | | |
|---|---|---|---|---|
| M3 c) | $S_B^*$ | 40 | 1,8 | 1,0 |
| | " | 35 | 1,9 | 1,1 |
| | " | 30 | 2,0 | 1,1 |

Fußnoten

a) M1

Binäre Mischung von Substanz II (x= 0,85) mit der Substanz (siehe DE-A 37 18 174) III:

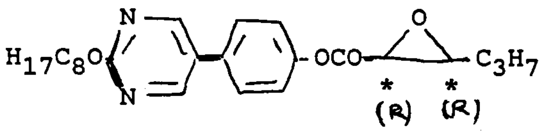 (III)

(Molenbruch x= 0,15).

Phasenfolge der Mischung: K 40 (unterkühlt 32) $S_B^*$ 68

$S_A^*$ 75-90 I

b) M2

Binäre Mischung aus IV (x= 0,88) und dem Dotierstoff V (x= 0,12):

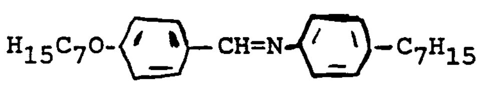 (IV)

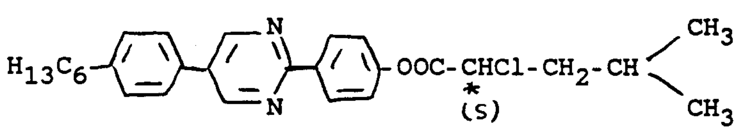 (V)

Klärpunkt der Mischung: 95°C

c) M3

Binäre Mischung aus IV (x= 0,72) und dem Dotierstoff VI (x= 0,28):

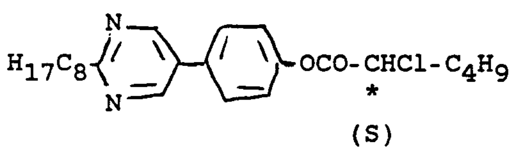 (VI)

Klärpunkt der Mischung: 66°C

Für die verwendeten Feldstärken von bis zu $4 \cdot 10^7$ V/m war sowohl in der $S_B^*$ als auch in der $S_E^*$ Phase der Neigungswinkel $\theta$ streng proportional der Feldstärke.

Bei der praktischen Verwertung des elektroklinen Effekts kann auf bekannte Techniken zurückgegriffen werden. So kann die feldabhängige Orientierung des Direktors $\hat{n}$ zusammen mit der optischen Anisotropie (Doppelbrechung) ausgenutzt werden.

Das Bauelement besteht aus einer planar orientierten $S_B^*$ oder $S_E^*$ Phase die sich zwischen zwei transparenten Elektroden und zwei gekreuzten Polarisatoren befindet. Solange kein Feld angelegt wird, befindet sich der Direktor $\hat{n}$ parallel zu einem der Polarisatoren, und es tritt kein Licht durch den zweiten Polarisator. Wird ein Feld angelegt, so neigt sich der Direktor $\hat{n}$, die Doppelbrechung wird wirksam, und das Element wird transparent.

Es ergeben sich Möglichkeiten zum Aufbau verschiedener Schalt- und Anzeigeelemente:

a. Elliptischer Polarisator

Bei diesem Effekt wird die Doppelbrechung des Flüssigkristalls ausgenutzt. Fällt linear polarisiertes Licht auf eine vorstehend beschriebene Zelle, die jedoch nicht mit Polarisatoren ausgestattet sein muß, so verläßt das Licht die Zelle elliptisch polarisiert. Spezialfälle sind zirkular oder wiederum linear polarisiert austretendes Licht. Durch den elektroklinen Effekt kann nun die Elliptizität kontinuierlich gesteuert werden.

b. Lichtmodulator ("birefringence mode")

Der unter a. beschriebene Fall kann unter Verwendung von Polarisatoren zur Lichtmodulation, d.h. zur Erzeugung elektrisch gesteuerter Intensitätsänderungen, benutzt werden. Die oben beschriebene Zelle konnte von uns bereits als Lichtmodulator eingesetzt werden.

Wenn man nicht-gekreuzte Polarisatoren verwendet, sondern einen variablen Winkel $\Psi$ zwischen Polarisator und Analysator einführt, so erhält man für die austretende Intensität I ($I_o$ ist die durch den Polarisator fallende Lichtintensität):

$$I(E) = I_o[\cos^2\Psi + \sin 4\theta(E) \cdot \sin 2(\Psi - 2\theta(E)) \cdot \sin^2(\pi \Delta n d/\lambda)]$$

$\theta(E)$ ist die feldinduzierte Neigung, d die Zellendicke, $\Delta n = n-n$ die optische Anisotropie, $\lambda$ die Vakuumwellenlänge des Lichtes.

Aus obenstehender Gleichung ergeben sich verschiedene Modulatoren, bei denen den extremen Feldstärken ($+E_{max}$ bzw. $-E_{max}$) gleiche (symmetrischer Modus) bzw. verschiedene Helligkeiten zukommen (unsymmetrischer Modus).

Ein Vorteil der höheren Empfindlichkeit ($d\theta/dE$) der höher geordneten smektischen Phasen ist eine erhöhte CMOS (± 15 V)-Kompatibilität.

c. Lichtmodulator ("guest-host mode")

Der Lichtmodulator kann auch mit Hilfe der Einlagerung dichroitisch absorbierender Moleküle gebaut werden. Dabei erübrigt sich einer der beiden Polarisatoren. Durch Anlegen eines elektrischen Feldes kann der Absorber zusammen mit der Flüssigkristallphase reorientiert und damit die Absorptionsachse gedreht werden. Die Absorber können auch IR-Chromophore enthalten.

d. Pulsschneider

Die unter a.-c. beschriebenen elektrooptischen Bauelemente können auch zur Erzeugung von zeitlich sich nicht wiederholenden Lichtimpulsen beliebiger Form verwendet werden.

Dazu kann man z.B. einen frei programmierbaren Pulsgenerator an eine oben beschriebenen Zelle schließen und unter Verwendung von Dauerstrich-Lichtquellen, wie cw-Lasern, beliebige Pulse schneiden.

e. Erzeugung einer Grauskala

Die lineare Kopplung des Neigungswinkels $\theta$ zum Feld E erlaubt die Erzeugung einer kontinuierlichen Grauskala für Bildschirme, Drucker u.ä..

Die Arbeitsweise dieses Bausteins ist wie unter a.-c. mit dem Unterschied, daß es sich um ein Element mit vielen kleinen Elektroden (sog. "pixels") handelt, die zusammen einen Grauschirm bilden.

f. Elektrisch ausrichtbare Phasen als Matrices für nicht-linear optische Materialien

Seit einiger Zeit ist bekannt, daß eine Reihe von organischen Verbindungen sehr große nicht-linear optische Koeffizienten besitzen (I. Zyss, J. Mol. Electron. 1, 25 (1985)).

Das Hauptproblem bei ihrer Anwendung besteht in der Ausrichtung, die entweder durch die Züchtung von Einkristallinen, durch die Langmuir-Blodgett-Technik oder durch die Einbettung in Flüssigkristall-Matrices geschehen kann.

Der elektrokline Effekt in LC-Phasen hoher optischer Güte kann zur elektrisch kontrollierten Feinabstimmung dieser Materialien (z.B. "phase matching" bei der Frequenzvervielfachung) verwendet werden.

g. Sensoren für elektrische Felder

Der elektrokline Effekt kann zum Nachweis eines unbekannten elektrischen Feldes ausgenutzt werden. Ein solcher Feldsensor besteht aus zwei Glasplatten ohne Elektroden zwischen denen sich ein planar orientierter $S_B^*$ oder $S_E^*$ -Flüssigkristall befindet. Gelangt dieser Sensor in ein elektrisches Feld, so läßt sich die Größe und die Richtung des Feldes mittels des elektroklinen Effektes nachweisen, indem man die Zelle zwischen gekreuzte Polarisatoren bringt oder auch den "guest-host" Modus verwendet.

Für solche Anwendungen ist eine hohe Empfindlichkeit ($d\theta/dE$) erforderlich, wie hier bei den höher geordneten Phasen gefunden wurde.

**Patentansprüche**

**1.** Verwendung von Verbindungen oder Gemischen von Verbindungen, die eine chirale, orthogonale, smektische Phase aufweisen, im Bereich dieser Phase als Anzeige- oder Schaltmedium in elektrooptischen Anzeige- oder Schaltelementen, die auf dem elektroklinen Effekt basieren, dadurch gekennzeichnet, daß die smektische Phase eine höher geordnete Phase als die $S_A^*$ -Phase ist.

**2.** Verwendung nach Anspruch 1, dadurch gekennzeichnet, daß die Phase eine $S_B^*$ oder $S_E^*$ Phase ist.

**3.** Verwendung von Verbindungen oder Gemischen von Verbindungen nach Anspruch 1 in elektrooptischen Anzeige- und Schaltelementen aus der Gruppe
a) elliptische Polarisatoren
b) Lichtmodulatoren in "birefringence mode"
c) Lichtmodulatoren in "guest-host mode"
d) Pulsschneider
e) Grauskalen für Bildschirme und Drucker
f) elektrisch gesteuerte Matrices für nicht-linear optische Materialien und
g) Sensoren für elektrische Felder.

**4.** Schalt- oder Anzeigeelement, das auf dem elektroklinen Effekt basiert, das als Schalt- oder Anzeigemedium eine Verbindung oder ein Gemisch von Verbindungen enthält, die im Betriebszustand des Schalt- oder Anzeigeelements in einer chiralen, orthogonalen, smektischen Phase vorliegen, dadurch gekennzeichnet, daß die smektische Phase eine höher geordnete Phase als die $S_A^*$ -Phase ist.

**5.** Schalt- oder Anzeigeelement, nach Anspruch 4, dadurch gekennzeichnet, daß die Phase eine $S_B^*$ oder $S_E^*$ Phase ist.

**Claims**

**1.** The use of compounds or mixtures of compounds which have a chiral, orthogonal, smectic phase in the range of said phase as indicating or switching medium in electro-optical indicating or switching

components which are based on the electroclinic effect, wherein the smectic phase is more highly ordered than the $S_A^*$ phase.

**2.** The use as claimed in claim 1, wherein the phase is an $S_B^*$ or $S_E^*$ phase.

**3.** The use of compounds or mixtures of compounds as claimed in claim 1 in electro-optical indicating and switching components from the group comprising
a) elliptical polarizers
b) light modulators in "birefringence mode"
c) light modulators in "guest-host mode"
d) pulse choppers
e) gray scales for display screens and printers
f) electrically controlled matrices for non-linear optical materials and
g) sensors for electric fields.

**4.** A switching or indicating component which is based on the electroclinic effect, comprising as switching or indicating medium a compound or a mixture of compounds which are present in a chiral, orthogonal, smectic phase in the operational state of the switching or indicating component, wherein the smectic phase is more highly ordered than the $S_A^*$ phase.

**5.** The switching or indicating component as claimed in claim 4, wherein the phase is an $S_B^*$ or $S_E^*$ phase.

**Revendications**

**1.** Utilisation de composés ou de mélanges de composés, qui présentent une phase chirale orthogonale smectique, dans le domaine de cette phase, à titre de moyen ou milieu d'affichage ou de commande dans des éléments électro-optiques d'affichage ou de commande, qui sont à base de l'effet électrocline, utilisation caractérisée en ce que la phase smectique est une phase plus fortement ordonnée que la phase $S_A^*$ .

**2.** Utilisation selon la revendication 1, caractérisée en ce que la phase est une phase $S_B^*$ ou $S_E^*$ .

**3.** Utilisation de composés ou de mélanges de composés selon la revendication 1 dans des éléments électro-optiques d'affichage ou de commande, choisis dans l'ensemble formé par :
a) des polariseurs elliptiques,
b) des modulateurs de lumière en "mode biréfringence",
c) des modulateurs de lumière en "guest-host mode" [mode invité-hôte],
d) des coupeurs ou hacheurs d'impulsions,
e) des échelles des gris pour des écrans et des imprimantes,
f) des matrices commandées électriquement pour des matériaux optiques non linéaires, et
g) des détecteurs de champs électriques.

**4.** Elément de commande ou d'affichage, qui est à base de l'effet électrocline, qui contient comme milieu ou moyen de commande ou d'affichage un composé ou un mélange de composés qui, lorsque l'élément de commande ou d'affichage est en état de fonctionnement, est ou sont présent(s) en une phase smectique chirale orthogonale, élément caractérisé en ce que la phase smectique est une phase plus fortement ordonnée que la phase $S_A^*$ .

**5.** Elément de commande ou d'affichage selon la revendication 4, caractérisé en ce que la phase est une phase $S_B^*$ ou $S_E^*$ .